Europäisches Patentamt

European Patent Office

Office européen des brevets

(19)

(11) Veröffentlichungsnummer: **0 007 983**
**A1**

# (12) EUROPÄISCHE PATENTANMELDUNG

(21) Anmeldenummer: 79101761.9

(22) Anmeldetag: 05.06.79

(51) Int. Cl.³: **C 07 C 85/26, C 07 C 87/123**

(30) Priorität: 04.08.78 DE 2834251

(43) Veröffentlichungstag der Anmeldung: 20.02.80
Patentblatt 80/4

(84) Benannte Vertragsstaaten: **BE FR GB IT NL**

(71) Anmelder: **CHEMISCHE WERKE HÜLS AG, - RSP Patente / PB 15 - Postfach 13 20, D-4370 Marl 1 (DE)**

(72) Erfinder: **Coenen, Alfred, Dr., Guido-Heiland-Strasse 3, D-4370 Marl (DE)**
Erfinder: **Kosswig, Kurt, Dr., Hoechster Strasse 10, D-4370 Marl (DE)**
Erfinder: **van Praun, Ferdinand, Dr., Am Schmöningsberg 8, D-4358 Haltern (DE)**
Erfinder: **Schüller, Hans-Peter, Brüderstrasse 16, D-4370 Marl (DE)**

(54) **Verfahren zur Reinigung von tertiären Aminen.**

(57) Verfahren zur Reinigung von vorzugsweise aus der Thermolyse von Aminhydrochloriden stammenden tertiären Aminen, indem man das zu reinigende Amin gegebenenfalls in einem organischen Lösemittel über Adsorptionsmittel leitet und aus dem Eluat gegebenenfalls das reine tertiäre Amin isoliert.

CHEMISCHE WERKE HÜLS AG     - 1 -     O.Z. 3035
- RSP PATENTE -

## Verfahren zur Reinigung von tertiären Aminen

Sowohl bei der Herstellung tertiärer Amine als auch bei deren thermischer Beanspruchung in manchen technischen Verfahren treten meist unerwünschte Nebenprodukte auf. Ihre vollständige Abtrennung mit Hilfe präparativer Methoden ist entweder umständlich oder nicht möglich.

Aufgabe der vorliegenden Erfindung war es daher, ein Verfahren zu entwickeln, das es gestattet, tertiäre Amine in einfacher und wirtschaftlicher Weise zu reinigen.

Diese Aufgabe wurde dadurch gelöst, daß man das zu reinigende Amin gegebenenfalls in einem organischen Lösemittel über Adsorptionsmittel leitet und aus dem Eluat gegebenenfalls das reine tertiäre Amin isoliert.

Die zu reinigenden tertiären Amine können z. B. beim Herstellungsprozeß oder bei der Thermolyse von tertiären Aminhydrochloriden, wie sie beispielsweise in der DE-OS 26 33 640 und der deutschen Patentanmeldung P 28 05 933.8 beschrieben ist, anfallen.

Bei der thermischen Spaltung von Aminhydrochloriden wurde nämlich festgestellt, daß sich tertiäre Amine in mehr oder weniger kurzer Zeit u. a. unter Bildung störender primärer und sekundärer Amine, die auch bei der Herstellung tertiärer Amine im allgemeinen den wesentlichen Teil der Ver-

unreinigungen darstellen, zersetzen. Diese Abbauamine bzw. deren Hydrochloride sind daher auch im Hinblick auf die Wirtschaftlichkeit solcher Verfahren vor der Rückführung der tertiären Amine zu entfernen.

Im allgemeinen geht man bei dem erfindungsgemäßen Verfahren so vor, daß man das zu reinigende tertiäre Amin vorzugsweise gelöst in einem geeigneten organischen Lösemittel über das Adsorptionsmittel leitet.

Geeignete Lösemittel sind solche, die ganz oder weitgehend unpolaren Charakter haben, wie z. B. geradkettige oder verzweigte aliphatische, cycloaliphatische, araliphatische oder aromatische Kohlenwasserstoffe. Typische Vertreter aus diesen Gruppen sind Hexan, Heptan, Octan, iso-Octan, Nonan, Decan, Undecan, Dodecan, Tetradecan, Cyclohexan, Methylcyclohexan, Decalin, Tetralin, Benzol, Toluol, Cumol, Xylole, Cymole, Ethylbenzol, p-tert.-Butyltoluol, Trimethylbenzole, 1.2.4-Triethylbenzol, 1.3.5-Triethylbenzol, tert.-Butyl-m-xylol, 1.2.3.4-Tetramethylbenzol, 1.2.3.5-Tetramethylbenzol, 3-Phenylpentan und Dodecylbenzol.

Die zweckmäßige Konzentration des tertiären Amins in der Lösung richtet sich nach der Art des Amins und der Menge der abzutrennenden Verunreinigungen und ist durch einige orientierende Versuche leicht zu ermitteln. Im allgemeinen arbeitet man mit einem Amin-Lösemittel-Verhältnis von 1 : 1 bis 1 : 10. Daneben kann weiteres Lösemittel als Elutionsmittel verwendet werden. Auch ist es möglich, bei hohen Konzentrationen an Verunreinigungen eine teilweise Vorabtrennung derselben durch Ausnutzung der verschiedenen Löslichkeiten im gewählten Lösemittel durchzuführen.

Für das erfindungsgemäße Verfahren geeignete Adsorptionsmittel sind Aluminiumoxide großer spezifischer Oberfläche (100 bis 400 m$^2$/g) in körniger, tablettierter oder gebrochener Form. Es hat sich gezeigt, daß die Basizität des Aluminiumoxids nicht entscheidend ist, es kann oberflächlich basisches, neutrales oder saures Aluminiumoxid eingesetzt werden. Wichtig dagegen ist die Aktivstufe. Brauchbar sind die Aktivstufen 1 bis 3, vorzugsweise die Aktivstufen 1 und 2. Als chromatographisches Adsorptionsmittel verwendetes Aluminiumoxid wird nach der Brockmann-Skala in 5 Aktivitätsstufen eingeteilt. Die nach Brockmann standartisierten Aluminiumoxide unterscheiden sich durch ihren Wassergehalt (in Gewichtsprozent): 1 (0 %), 2 (3 %), 3 (4,5 %), 4 (9,5 %), 5 (15 %) (O. A. Neumüller, Römpps Chemie-Lexikon, Seite 427 (1972); weitere Literatur dort).

Weitere geeignete Adsorptionsmittel sind Kieselgele mit spezifischen Oberflächen von 500 bis 700 m$^2$/g, silanisierte Kieselgele (300 bis 400 m$^2$/g) sowie Molekularsiebe vom Calciumzeolithtyp (500 bis 600 m$^2$/g). In den Kieselgelen liegt die Kieselsäure in Form hochkondensierter Polykieselsäuren mit oberflächenreicher Blattstruktur vor. Kieselgele, deren hydrophile Oberflächen durch Umsetzung mit Chlorsilanen hydrophobiert wurden, werden als silanisierte Kieselgele bezeichnet.
Die spezifische Oberfläche wird nach BET (S. Brunauer, P. H. Emmett, E. Teller, J. Am. Chem. Soc. 60, 309 (1938)) bestimmt.

Im allgemeinen führt man das erfindungsgemäße Verfahren bei Raumtemperatur (15 bis 30 °C) durch, jedoch kann es auch zweckmäßig sein, bei höheren Temperaturen zu arbeiten.

Das anfallende Eluat kann entweder direkt oder nach beliebiger Einkonzentrierung bis zur Lösemittelfreiheit verwendet werden. Das Adsorptionsmittel läßt sich ohne Schwierigkeiten mit Hilfe polarer Lösemittel regenerieren. Bevorzugt werden nach dem erfindungsgemäßen Verfahren tertiäre Amine mit insgesamt 6 bis 54 Kohlenstoffatomen in den Alkylgruppen gereinigt, die aus der Thermolyse von

0007983

tertiären Aminhydrochloriden stammen und die anschließend ohne Abtrennung des Lösemittels rezirkuliert werden. Der Reinheitsgrad des gereinigten tertiären Amins sollte möglichst über 99 %, vorzugsweise über 99,5 % und besonders bevorzugt über 99,9 % liegen.

Das erfindungsgemäße Verfahren wird durch die nachfolgenden Beispiele erläutert.

Beispiel 1

Auf eine Adsorptionssäule (Durchmesser 22 mm), die mit 225 ml saurem Aluminiumoxid (Aktivstufe 1, spezifische Oberfläche 200 $m^2$/g) beschickt ist, wird ein Gemisch aus 45 g Tri-(2-ethylhexyl)-amin und 5 g Di-(2-ethylhexyl)-aminhydrochlorid, gelöst in 50 g Xylol, aufgegeben. Als Elutionsmittel werden 250 ml Xylol eingesetzt. Das abfließende Eluat enthält 44,5 g gaschromatographisch reines tert. Amin. 4,6 g des sek. Aminhydrochlorids werden durch Waschen des Adsorptionsmittels mit einer Aceton-Methanolmischung (1 : 1) isoliert. Das polare Lösemittelgemisch wird wieder gegen Xylol ausgetauscht; eine erneute Auftrennung eines Amingemisches ist jetzt ohne weitere Regenerierung des Adsorptionsmittels möglich.

Beispiel 2

Eine Mischung aus 1 492,5 g Tri-(2-ethylhexyl)-amin und 7,5 g Di-(2-ethylhexyl)-aminhydrochlorid, gelöst in 1 500 g Xylol, wird mit 225 ml saurem Aluminiumoxid (Aktivstufe 1, spezifische Oberfläche 200 $m^2$/g) in einer Adsorptionssäule gemäß Beispiel 1 aufgetrennt, ohne daß zusätzliches Elutionsmittel verwendet wird. Es werden 1 270 g gaschromatographisch reines tert. Amin erhalten.

Beispiel 3

Eine Mischung aus 90 g Tri-(2-ethylhexyl)-amin, 10 g Di-(2-ethylhexyl)-amin und 1 g 2-Ethylhexylamin, gelöst in 100 g Xylol, wird gemäß Versuchsbeschreibung in Beispiel 2 aufgetrennt. Bis zum Auftreten von sek. und prim. Amin im Eluat werden 81 g gaschromatographisch reines tert. Amin isoliert.

Beispiel 4

Eine Mischung aus 200 g Tri-(2-ethylhexyl)-amin und 4 g Di-(2-ethylhexyl)-amin, gelöst in 200 g Xylol, wird an 225 ml silanisiertem Kieselgel (spezifische Oberfläche 335 $m^2$/g) gemäß Versuchsbeschreibung in Beispiel 2 aufgetrennt. Bis zum Auftreten von sek. Amin im Eluat werden 115 g gaschromatographisch reines tert. Amin erhalten.

Beispiel 5

Eine Mischung aus 200 g Tri-(2-ethylhexyl)-amin und 4 g Di-(2-ethylhexyl)-amin, gelöst in 200 g Xylol, wird an 225 ml Kieselgel der spezifischen Oberfläche von 700 $m^2$/g gemäß Versuchsbeschreibung in Beispiel 2 aufgetrennt. Bis zum Auftreten von sek. Amin im Eluat werden 100 g gaschromatographisch reines tert. Amin erhalten.

Beispiel 6

Eine Mischung aus 200 g Tri-(2-ethylhexyl)-amin und 4 g Di-(2-ethylhexyl)-amin, gelöst in 200 g Xylol, wird an 225 ml Kieselgel der spezifischen Oberfläche von 500 $m^2$/g gemäß Versuchsbeschreibung in Beispiel 2 aufgetrennt. Bis zum Auftreten von sek. Amin im Eluat werden 60 g gaschromatographisch reines tert. Amin erhalten.

Beispiel 7

Eine Mischung aus 500 g Tri-(2-ethylhexyl)-amin und 5 g Di-(2-ethylhexyl)-amin, gelöst in 500 g Xylol, wird an 225 ml Calciumzeolith der spezifischen Oberfläche von 550 $m^2$/g ge-

mäß Versuchsbeschreibung in Beispiel 2 aufgetrennt. Bis zum Auftreten von sek. Amin im Eluat werden 250 g gaschromatographisch reines tert. Amin isoliert.

Beispiel 8

Eine Mischung aus 1 492,5 g Trioctylamin und 7,5 g Dioctylamin, gelöst in 1 500 g Xylol, wird an 225 ml saurem Aluminiumoxid (Aktivstufe 1, spezifische Oberfläche 200 $m^2$/g) gemäß Versuchsbeschreibung in Beispiel 2 aufgetrennt. Bis zum Auftreten von sek. Amin im Eluat werden 1 190 g gaschromatographisch reines tert. Amin isoliert.

Beispiel 9

Eine Mischung aus 180 g Triethylamin und 20 g Diethylamin, gelöst in 200 g Xylol, wird an 225 ml saurem Aluminiumoxid (Aktivstufe 1, spezifische Oberfläche 200 $m^2$/g) gemäß Versuchsbeschreibung in Beispiel 2 aufgetrennt. Bis zum Auftreten von sek. Amin im Eluat werden 110 g gaschromatographisch reines tert. Amin isoliert.

Beispiel 10

Eine Mischung aus 180 g Trioctylamin und 20 g Dioctylamin wird an 225 ml saurem Aluminiumoxid (Aktivstufe 1, spezifische Oberfläche 200 $m^2$/g) gemäß Versuchsbeschreibung in Beispiel 2 aufgetrennt. Bis zum Auftreten von sek. Amin am Auslauf der Adsorptionssäule werden 105 g gaschromatographisch reines tert. Amin isoliert.

Beispiele 11 und 12

Ein Gemisch aus 100 g Trilaurylamin und 500 g Dilaurylamin bzw. 100 g Tristearylamin und 500 g Distearylamin wird zur Vortrennung in Xylol aufgeschlämmt. Hierbei gehen die tert. Amine quantitativ in Lösung, während die sek. Amine sich höchstens bis zu 5 % lösen.

Zur vollständigen Abtrennung der sek. Amine wird das Filtrat mit 225 ml saurem Aluminiumoxid der spezifischen Oberfläche von 200 $m^2/g$ in beschriebener Weise behandelt. Das jeweilige Eluat enthält die tert. Amine, die laut dünnschichtchromatographischer Analyse frei von sek. Aminen sind.

## Beispiel 13

Durch eine Adsorptionssäule (Durchmesser 100 mm), beschickt mit 8 kg saurem Aluminiumoxid (Aktivstufe 1, spezifische Oberfläche 200 $m^2/g$), wird kontinuierlich pro Stunde ein Gemisch aus 150 g Trioctylamin und 0,5 g Dioctylamin, gelöst in 600 g Dodecan, das aus einem Versuch zur thermischen Spaltung von Aminhydrochloriden stammt, geleitet. Das ablaufende Eluat bleibt ca. 800 Stunden frei von sek. Amin.

Patentansprüche:

1. Verfahren zur Reinigung von tertiären Aminen,
   dadurch gekennzeichnet,
   daß man das zu reinigende Amin gegebenenfalls in einem
   organischen Lösemittel über Adsorptionsmittel leitet
   und aus dem Eluat gegebenenfalls das reine tertiäre
   Amin isoliert.

2. Verfahren nach Anspruch 1,
   dadurch gekennzeichnet,
   daß das zu reinigende tertiäre Amin aus der Thermolyse
   von tertiären Aminhydrochloriden stammt.

3. Verfahren nach den Ansprüchen 1 und 2,
   dadurch gekennzeichnet,
   daß man als Adsorptionsmittel Aluminiumoxid mit einer
   spezifischen Oberfläche von 100 bis 400 $m^2$/g, Kieselgel
   mit einer spezifischen Oberfläche von 500 bis 700 $m^2$/g,
   silanisiertes Kieselgel mit einer spezifischen Oberfläche von 300 bis 400 $m^2$/g oder Calciumzeolith mit einer
   spezifischen Oberfläche von 500 bis 600 $m^2$/g, jeweils
   gemessen nach BET, einsetzt.

0007983

Neuer Patentanspruch:

Verfahren zur Reinigung von tertiären Aminen,
dadurch gekennzeichnet,
daß man tertiäre Amine, die aus der Thermolyse von tertiären Aminhydrochloriden stammen, gegebenenfalls gelöst in einem organischen Lösemittel, über Aluminiumoxid mit einer spezifischen Oberfläche von 100 bis 400 $m^2$/g, Kieselgel mit einer spezifischen Oberfläche von 500 bis 700 $m^2$/g, silanisiertes Kieselgel mit einer spezifischen Oberfläche von 300 bis 400 $m^2$/g oder Calciumzeolith mit einer spezifischen Oberfläche von 500 bis 600 $m^2$/g, jeweils gemessen nach BET, leitet und aus dem Ablauf gegebenenfalls das reine tertiäre Amin isoliert.

Europäisches
Patentamt

**EUROPÄISCHER RECHERCHENBERICHT**

0007983
Nummer der Anmeldung

EP 79 101 761.9

| | EINSCHLÄGIGE DOKUMENTE | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| X | <u>GB - A - 1 096 707</u> (COMMISSARIAT A L'ENERGIE ATOMIQUE) <br> * Anspruch 1; Seite 1, Spalte 1, Zeilen 36 bis 38 <br> -- | 1,3 | C 07 C 85/26 <br> C 07 C 87/123 |
| A | <u>US - A - 3 975 387</u> (TEXACO) <br> ---- | | |

RECHERCHIERTE SACHGEBIETE (Int. Cl.²)

C 07 C 85/26

C 07 C 87/123

KATEGORIE DER GENANNTEN DOKUMENTE

X: von besonderer Bedeutung

A: technologischer Hintergrund

O: nichtschriftliche Offenbarung

P: Zwischenliteratur

T: der Erfindung zugrunde liegende Theorien oder Grundsatze

E: kollidierende Anmeldung

D: in der Anmeldung angeführtes Dokument

L: aus andern Gründen angeführtes Dokument

&: Mitglied der gleichen Patent-familie, übereinstimmendes Dokument

X | Der vorliegende Recherchenbericht wurde für alle Patentanspruche erstellt.

| Recherchenort | Abschlußdatum der Recherche | Prüfer |
|---|---|---|
| Berlin | 10-08-1979 | KAPTEYN |

EPA form 1503.1 06.78